Europäisches Patentamt

European Patent Office

Office européen des brevets

(19)

(11) Veröffentlichungsnummer: **0 210 480**
**B1**

(12) **EUROPÄISCHE PATENTSCHRIFT**

(45) Veröffentlichungstag der Patentschrift:
07.06.89

(51) Int. Cl.⁴: **C01B 7/07**

(21) Anmeldenummer: **86109312.8**

(22) Anmeldetag: **08.07.86**

(54) Verfahren zur Reinigung von Chlorwasserstoffgas.

(30) Priorität: **26.07.85 DE 3526801**

(43) Veröffentlichungstag der Anmeldung:
**04.02.87 Patentblatt 87/6**

(45) Bekanntmachung des Hinweises auf die Patenterteilung:
**07.06.89 Patentblatt 89/23**

(84) Benannte Vertragsstaaten:
**DE FR GB NL SE**

(56) Entgegenhaltungen:
**DE-B- 2 522 286**
**GB-A- 928 179**

(73) Patentinhaber: **HOECHST AKTIENGESELLSCHAFT,**
**Postfach 80 03 20, D-6230 Frankfurt am Main 80(DE)**

(72) Erfinder: **Müller, Heinz, Breslauer Strasse 66,**
**D-5040 Brühl(DE)**
Erfinder: **Lohmar, Elmar, Dr., Drachenfelsstrasse 47,**
**D-5000 Köln(DE)**
Erfinder: **Scholz, Harald, Dr., Am Beissel 8,**
**D-5042 Erftstadt(DE)**

## Beschreibung

Die Erfindung betrifft ein Verfahren zur Reinigung eines Chlorwasserstoffgases, wie es bei der Herstellung von Chloressigsäuren durch katalytische Chlorierung von Essigsäure mit Chlorgas in Gegenwart von Essigsäureanhydrid und/oder Acetylchlorid im kontinuierlichen Betrieb als Nebenprodukt erhalten wird.

Nach Ullmans Encyklopädie der technischen Chemie, 3. Auflage, Band 5, Seiten 390 und 391, ist es bekannt, Chloressigsäuren durch Chlorierung von mindestens 98 %iger Essigsäure mit Chlor unter Zusatz von Essigsäureanhydrid und/oder Acetylchlorid als Katalysator bei einer Temperatur von 85°C oder höher kontinuierlich herzustellen. Die Verwertung des hierbei als Nebenprodukt anfallenden Chlorwasserstoffes verlangt die Entfernung der kondensierbaren Anteile, die als wertvolle Beschleuniger zweckmäßig in die Essigsäurechlorierung zurückgeführt werden. Zu diesem Zweck werden die abziehenden Gase mit Frischprodukt im Gegenstrom gewaschen und/oder durch Tiefkühlung von kondensierbaren Bestandteilen befreit.

Eine derartige Verfahrensweise zur Herstellung von Chloressigsäuren wird auch in der deutschen Offenlegungsschrift 19 19 476 beschrieben, wobei zur Rückgewinnung von Acetylchlorid und gegebenenfalls weiterer Abgasprodukte des Reaktors das Reaktorabgas auf eine Temperatur von mindestens etwa 20°C abgekühlt und außerdem im Gegenstrom mit dem zu chlorierenden Gemisch aus Essigsäure, Acetanhydrid und/oder Acetylchlorid gewaschen wird.

Das auf vorbeschriebene Weise gereinigte Chlorwasserstoffgas enthält noch zwischen 0,6 und 3 Vol.-% Carbonsäurechloride in Form von vorwiegend Acetylchlorid und gegebenenfalls etwas Chloracetylchlorid. Erfahrungsgemäß ist ein derartig verunreinigtes Rohchlorwasserstoffgas außerordentlich korrosiv, besonders wenn das Gas zur Kondensation der kondensierbaren Anteile komprimiert wird. Es war deshalb bisher nicht möglich, ein solches Rohchlorwasserstoffgas auf diesem Wege in herkömmlichen Vorrichtungen in dem Maße zu reinigen, daß das gereinigte Chlorwasserstoffgas für weitere chemische Reaktionen verwendet werden kann. Man hat sich daher damit begnügt, das Rohchlorwasserstoffgas in Wasser bzw. Salzsäure zu absorbieren. Die dabei anfallende Salzsäure ist mit Essigsäure verunreinigt. Eine destillative Trennung des Salzsäure/Essigsäure-Gemisches bereitet aufgrund des geringen Siedepunktunterschiedes von Essigsäure und dem azeotropischen Gemisch von Chlorwasserstoff und Wasser Schwierigkeiten und ist außerdem unwirtschaftlich. Vielfach mußte deshalb die mit Essigsäure verunreinigte Salzsäure verworfen werden.

In der DE 25 22 286 C3 wird ein Verfahren zur Reinigung des Chlorwasserstoffgases, wie es bei der Herstellung von Chloressigsäure durch Chlorierung von Essigsäure anfällt, beschrieben, bei dem das verunreinigte Chlorwasserstoffgas im Gegenstrom mit einer Waschflüssigkeit behandelt wird, welche aus etwa 20-80 Gew% $H_2SO_4$, etwa 15-60 Gew% Essigsäure und etwa 5-50 Gew% Wasser besteht.

Nachteilig bei diesem Reinigungsverfahren ist, daß hierbei eine im Kreislauf geführte verdünnte Schwefelsäure anfällt, die größere Mengen Essigsäure enthält und außerdem durch gelöste Salzsäure äußerst aggressiv und daher schwer zu handhaben ist. Nur durch einen aufwendigen Reinigungsprozeß konnte hieraus die Essigsäure zurückgewonnen werden.

Es ist daher die Aufgabe gestellt, ein Reinigungsverfahren zu finden, das eine wirtschaftliche Reinigung des Chlorwasserstoffgases, wie es bei der Herstellung von Chloressigsäuren durch Chlorierung von Essigsäure anfällt, ermöglicht, unter Bildung nur solcher Stoffe, die ohne aufwendige Reinigungsschritte wieder verwendet werden können.

Das erfindungsgemäße Verfahren zur Reinigung eines bei der Herstellung von Chloressigsäuren durch katalytische Chlorierung von Essigsäure mit Chlorgas in Gegenwart von Essigsäureanhydrid und/oder Acetylchlorid als Nebenprodukt anfallenden Chlorwasserstoffgases, welches nach Vorreinigung noch etwa 0,1 - 3 Vol% Acetylchlorid und bis etwa 0,1 Vol% Chloracetylchlorid als Verunreinigungen enthält, durch zwei hintereinander geschaltete, im Gegenstrom betriebene, unterteilte Waschzonen, ist dadurch gekennzeichnet, daß in der ersten Waschzone das Chlorwasserstoffgas mit konzentrierter Salzsäure gewaschen wird und in der zweiten Waschzone das vorgereinigte Chlorwasserstoffgas mit konzentrierter Schwefelsäure nachgereinigt wird.

Zweckmäßig sind die beiden Waschzonen jeweils in einen unteren und einen oberen Teil aufgeteilt.

Das erfindungsgemäße Verfahren wird bevorzugt so durchgeführt, daß das zu reinigende Chlorwasserstoffgas zunächst im unteren Teil der ersten Waschzone mit konzentrierter Salzsäure, die bis 15 Gew% Essigsäure, vorzugsweise bis 7-10 Gew% Essigsäure, enthalten kann, bei einer Temperatur von 10 - 50°C, vorzugsweise 15 - 25°C, behandelt wird, und danach das Chlorwasserstoffgas im oberen Teil der ersten Waschzone mit konzentrierter Salzsäure bei einer Temperatur von 5 - 40°C, vorzugsweise 18 bis 20°C, gewaschen wird.

Es hat sich als vorteilhaft erwiesen, wenn der untere Teil der ersten Waschzone mit 1 - 5 l, vorzugsweise 2 - 3 l Waschflüssigkeit, bestehend aus konzentrierter Salzsäure und eventuell Essigsäure, pro m³ Chlorwasserstoffgas - bezogen auf Normalbedingungen - beaufschlagt und der obere Teil der ersten Waschzone mit 0,05 - 1 l, vorzugsweise 0,1 l konzentrierter Salzsäure pro m³ Chlorwasserstoffgas - bezogen auf Normalbedingungen - beaufschlagt wird.

Eine vorteilhafte Ausgestaltung des Verfahrens ist gegeben, wenn das Chlorwasserstoffgas aus dem oberen Teil der ersten Waschzone zunächst in den unteren Teil der zweiten Waschzone mit einer Schwefelsäure, die bis zu 15 Gew%, vorzugsweise bis zu 10 Gew% Wasser enthält, bei einer Temperatur von 10 - 40°C, vorzugsweise 15 - 20°C, gewaschen wird und danach das Chlorwasserstoffgas im oberen Teil der zweiten Waschzone mit konzentrierter Schwefelsäure bei einer Temperatur von 10 - 30°C, vorzugsweise 18 - 20°C, gewaschen wird.

Eine gute Reinigung wird erzielt, wenn der untere Teil der zweiten Waschzone mit 2 - 10 l,vorzugsweise 5 - 6 l Schwefelsäure, die bis zu 15 Gew%, vorzugsweise bis zu 10 Gew% Wasser enthält, pro m³ Chlorwasserstoffgas - bezogen auf Normalbedingungen - beaufschlagt wird.

Zweckmäßig ist, wenn der obere Teil der zweiten Waschzone mit 0,01 - 0,5 l, vorzugsweise 0,02 - 0,1 l konzentrierter Schwefelsäure pro m³ Chlorwasserstoffgas - bezogen auf Normalbedingungen - beaufschlagt wird.

Mit dem Verfahren der Erfindung kann nunmehr das Chlorwasserstoffgas, wie es bei der Herstellung von Chloressigsäure durch Chlorierung anfällt, so gereinigt werden, daß ein Chlorwasserstoffgas erhalten wird, das weniger als 10 ppm Wasser und weniger als 5 ppm Essigsäure enthält.

Die aus der ersten Waschzone anfallende konzentrierte Salzsäure mit bis zu 15 Gew% Essigsäure kann quantitativ in die Monochloressigsäureherstellung rückgeführt werden, wo diese Salzsäure zur Hydrolyse von Säurechloriden bzw. -anhydriden genutzt wird.

Die aus der zweiten Waschzone anfallende Schwefelsäure mit bis zu 15 Gew% Wasser enthält weniger als 0,5 Gew% Essigsäure und weniger als 0,1 Gew% Chlorwasserstoff. Durch Ausblasen mit Luft kann der Chlorwasserstoffgehalt der Schwefelsäure auf < 10 ppm abgereichert und kann diese beispielsweise direkt zum Aufschluß von Phosphaterzen eingesetzt werden.

Das Verfahren der Erfindung wird anhand eines Fließschemas beispielhaft erläutert:

Aus der Monochloressigsäureherstellung werden über die Leitung 13 1800 Nm³/h Chlorwasserstoffgas mit 0,15 Vol% Acetylchlorid, 0,01 Vol% Chloracetylchlorid und 0,1 Vol% Essigsäure als Verunreinigungen in die erste Waschzone 1 eingeführt. Über den Flüssigkeitskreislauf 14 mit der Pumpe 11 werden 4 m³/h Waschlösung, enthaltend 39,8 Gew% Chlorwasserstoff und 8,9 Gew% Essigsäure, im unteren Teil 3 der ersten Waschzone 1 umgewälzt. Mittels Solekühler 15 wird die Waschlösung auf 17°C eingestellt. Der untere Teil der ersten Waschzone ist ein Kolonnenschuß mit einem lichten Durchmesser von 700 mm und 10 Glockenböden. Über die Leitung 17 wird konzentrierte Salzsäure aus dem Vorrat 8 in den oberen Teil 4 der ersten Waschzone 1 in einer Menge von 180 l/h mit einer Temperatur von 20°C eingebracht. Der obere Teil der ersten Waschzone ist ein Kolonnenschuß mit einem lichten Durchmesser von 700 mm und 10 Glockenböden.

Über die Leitung 16 wird die verbrauchte Waschlösung in den Vorrat 7 (Monochloressigsäureherstellung) abgelassen.

Das vorgereinigte Chlorwasserstoffgas verläßt die erste Waschzone über die Leitung 18 und gelangt in die zweite Waschzone 2. Im unteren Teil 5 der zweiten Waschzone 2 wird das Chlorwasserstoffgas über den Flüssigkeitskreislauf 19 mit der Pumpe 12 mit 10 m³/h Schwefelsäure, die 7,9 Gew% Wasser enthält, gewaschen. Der untere Teil der zweiten Waschzone ist ein Kolonnenschuß mit einem lichten Durchmesser von 700 mm und 10 Glockenböden.

Der Flüssigkeitskreislauf 19 wird mittels Solekühler 21 auf einer Temperatur von 16°C gehalten. Der obere Teil 6 der zweiten Waschzone 2 ist ein Kolonnenschuß mit einem lichten Durchmesser von 700 mm und 10 Glockenböden und wird aus dem Vorrat 10 über die Leitung 23 mit 60 l/h konzentrierter Schwefelsäure beaufschlagt. Das gereinigte Chlorwasserstoffgas verläßt die zweite Waschzone 2 über Leitung 20. Als Verunreinigung enthält das Chlorwasserstoffgas < 10 ppm Wasser und < 5 ppm Essigsäure.

Die in den Vorrat 9 über Leitung 22 abgelassene verbrauchte Schwefelsäure enthält neben 7,9 Gew% Wasser noch < 0,5 Gew% Essigsäure und 0,09 Gew% Chlorwasserstoff.

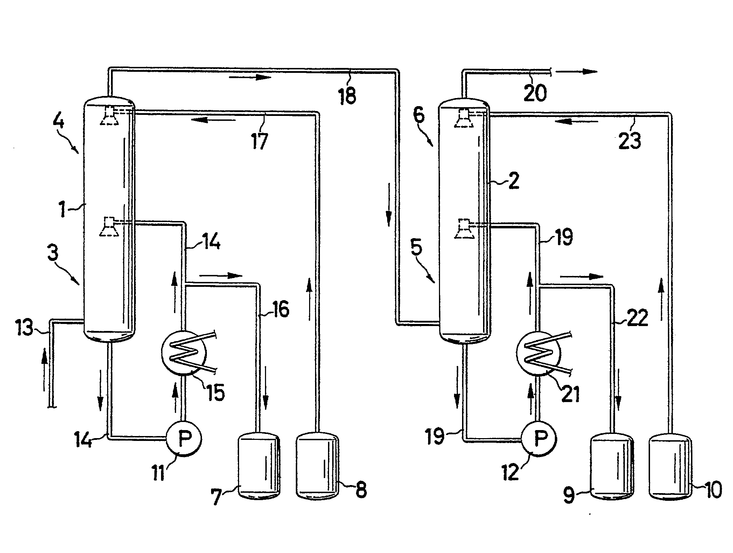

## Patentansprüche

1. Verfahren zur Reinigung eines bei der Herstellung von Chloressigsäuren durch katalytische Chlorierung von Essigsäure mit Chlorgas in Gegenwart von Essigsäureanhydrid und/oder Acetylchlorid als Nebenprodukt anfallenden Chlorwasserstoffgases, welches nach Vorreinigung noch etwa 0,1 - 3 Vol% Acetylchlorid und bis etwa 0,1 Vol% Chloracetylchlorid als Verunreinigungen enthält, durch zwei hintereinander geschaltete, im Gegenstrom betriebene, unterteilte Waschzonen, dadurch gekennzeichnet, daß in einer ersten Waschzone das Chlorwasserstoffgas mit konzentrierter Salzsäure gewaschen wird und in einer zweiten Waschzone das vorgereinigte Chlorwasserstoffgas mit konzentrierter Schwefelsäure nachgereinigt wird.

2. Verfahren nach Anspruch 1, dadurch gekennzeichnet, daß das zu reinigende Chlorwasserstoffgas zunächst im unteren Teil der ersten Waschzone mit konzentrierter Salzsäure, die bis 15 Gew% Essigsäure, vorzugsweise bis 7-10 Gew% Essigsäure, enthalten kann, bei einer Temperatur von 10 - 50°C, vorzugsweise 15-25°C, behandelt wird und danach das Chlorwasserstoffgas im oberen Teil der ersten Waschzone mit konzentrierter Salzsäure bei einer Temperatur von 5 - 40°C, vorzugsweise 18 bis 20°C, gewaschen wird.

3. Verfahren nach Anspruch 2, dadurch gekennzeichnet, daß der untere Teil der ersten Waschzone mit 1 - 5 l, vorzugsweise 2 - 3 l, Waschflüssigkeit, bestehend aus konzentrierter Salzsäure und eventuell Essigsäure, pro m³ Chlorwasserstoffgas - bezogen auf Normalbedingungen - beaufschlagt wird.

4. Verfahren nach Anspruch 2, dadurch gekennzeichnet, daß der obere Teil der ersten Waschzone mit 0,05 - 1 l, vorzugsweise 0,1 l konzentrierter Salzsäure pro m³ Chlorwasserstoffgas - bezogen auf Normelbedingungen - beaufschlagt wird.

5. Verfahren nach Anspruch 2, dadurch gekennzeichnet, daß das Chlorwasserstoffgas aus dem oberen Teil der ersten Waschzone zunächst im unteren Teil der zweiten Waschzone mit einer Schwefelsäure, die bis zu 15 Gew%, vorzugsweise bis zu 10 Gew% Wasser enthält, bei einer Temperatur von 10 - 40°C, vorzugsweise 15 -20°C, gewaschen wird und danach das Chlorwasserstoffgas im oberen Teil der zweiten Waschzone mit konzentrierter Schwefelsäure bei einer Temperatur von 10 - 30°C, vorzugsweise 18 - 20°C, gewaschen wird.

6. Verfahren nach Anspruch 5, dadurch gekennzeichnet, daß der untere Teil der zweiten Waschzone mit 2 - 10 l, vorzugsweise 5 - 6 l Schwefelsäure, die bis zu 15 Gew% , vorzugsweise bis zu 10 Gew% Wasser enthält, pro m³ Chlorwasserstoffgas - bezogen auf Normalbedingungen - beaufschlagt wird.

7. Verfahren nach Anspruch 5, dadurch gekennzeichnet, daß der obere Teil der zweiten Waschzone mit 0,01 - 0,5 l, vorzugsweise 0,02 - 0,1 l konzentrierter Schwefelsäure pro m³ Chlorwasserstoffgas - bezogen auf Normalbedingungen - beaufschlagt wird.

## Claims

1. A process for purifying hydrogen chloride gas which arises as a by-product in the manufacture of chloroacetic acids by catalytic chlorination of acetic acid with chloride gas in the presence of acetic anhydride and/or acetyl chloride and which, after prepurification, still contains about 0.1–3% by volume of acetyl chloride and up to about 0.1% by volume of chloroacetyl chloride as impurities, by means of two subdivided scrubbing zones arranged in series and operated in counter-current, which comprises scrubbing the hydrogen chloride gas with concentrated hydrochloric acid in a first scrubbing zone and further purifying the prepurified hydrogen chloride gas with concentrated sulfuric acid in the second scrubbing zone.

2. The process as claimed in claim 1, wherein the hydrogen chloride gas to be purified is first treated in the lower part of the first scrubbing zone with concentrated hydrochloride acid, which can contain up to 15% by weight of acetic acid, preferably up to 7–10% by weight of acetic acid, at a temperature of 10–50°C, preferably 15–25°C, and then scrubbing the hydrogen chloride gas in the upper part of the first scrubbing zone with concentrated hydrochlorid acid at a temperature of 5–40°C, preferably 18 to 20°C.

3. The process as claimed in claim 2, wherein 1–5 l, preferably 2–3l, of scrubbing liquid composed of concentrated hydrochloric acid and possibly acetic acid are charged to the lower part of the first scrubbing zone per m³ of hydrogen chloride gas – relative to standard conditions.

4. The process as claimed in claim 2, wherein 0.05–1 l, preferably 0.1 l, of concentrated hydrochlorid acid is charged to the upper part of the first scrubbing zone per m³ of hydrogen chloride gas – relative to standard conditions.

5. The process as claimed in claim 2, wherein the hydrogen chloride gas from the upper part of the first scrubbing zone is first scrubbed in the lower part of the second scrubbing zone with a sulfuric acid which contains up to 15% by weight, preferably up to 10% by weight, of water at a temperature of 10–40°C, preferably 15–20°C, and the hydrogen chloride gas is then scrubbed in the upper part of the second scrubbing zone with concentrated sulfuric acid at a temperature of 10–30°C, preferably 18–20°C.

4. The process as claimed in claim 5, wherein 2–10 l, preferably 5–6 l of sulfuric acid which contains up to 15%, preferably up to 10 by weight, of water is charged to the lower part of the second scrubbing zone per m³ of hydrogen chloride gas – relative to standard conditions.

7. The process as claimed in claim 5, wherein 0.01–0.5 l, preferably 0.02–0.1 l, of concentrated sulfuric acid is charged to the upper part of the second scrubbing zone per m³ of hydrogen chloride gas – relative to standard conditions.

## Revendications

1. Procédé pour la purification d'un gaz chlorhydrique obtenu comme sous-produit dans la fabrication d'acides chloroacétiques par chloration catalytique de l'acide acétique par le chlore gazeux en présence d'anhydride acétique et/ou de chlorure d'acétyle, qui, après une purification préalable, contient encore comme impuretés environ 0,1–3% en volume de chlorure d'acétyle et jusqu'à environ 0,1% en volume de chlorure de chloroacétyle, à travers deux zones de lavage subdivisées, fonctionnant à contrecourant, branchées l'une à la suite de l'autre, caractérisé en ce que le gaz chlorhydrique est lavé dans une première zone de lavage par l'acide chlorhydrique concentré et le gaz chlorhydrique préalablement purifié est encore purifié dans une seconde zone de lavage par l'acide sulfurique concentré.

2. Procédé selon la revendication 1, caractérisé en ce que le gaz chlorhydrique à purifier est d'abord traité dans la partie inférieure de la première zone de lavage par l'acide chlorhydrique concentré qui peut contenir jusqu'à 15% en poids d'acide acétique, de préférence jusqu'à 7–10% en poids d'acide acétique, à une température de 10–50°C, de préférence 15–25°C, et ensuite le gaz chlorhydrique est lavé dans la partie supérieure de la première zone de lavage par l'acide chlorhydrique concentré à une température de 50–40°C, de préférence 18 à 20°C.

3. Procédé selon la revendication 2, caractérisé en ce que la partie inférieure de la première zone de lavage est chargée avec 1–5 l, de préférence 2–3 l, de liquide de lavage, consistant en acide chlorhydrique concentré et éventuellement en acide acétique, par m³ de gaz chlorhydrique – ramené aux conditions normales.

4. Procédé selon la revendication 2, caractérisé en ce que la partie supérieure de la première zone de lavage est chargée avec 0,05–1 l, de préférence 0,1 l d'acide chlorhydrique, par m³ de gaz chlorhydrique – ramené aux conditions normales.

5. Procédé selon la revendication 2, caractérisé en ce que le gaz chlorhydrique provenant de la partie supérieure de la première zone de lavage est d'abord lavé dans la partie inférieure de la seconde zone de lavage par un acide sulfurique qui contient jusqu'à 15% en poids, de préférence jusqu'à 10% en poids d'eau, à une température de 10–40°C de préférence 15–20°C, et ensuite le gaz chlorhydrique est lavé dans la partie supérieure de la seconde zone de lavage par l'acide sulfurique concentré à une température de 10–30°C, de préférence 18–20°C.

6. Procédé selon la revendication 5, caractérisé en ce que la partie inférieure de la seconde zone de lavage est chargée avec 2–10 l, de préférence 5–6 l d'acide sulfurique, qui contient jusqu'à 15% en poids, de préférence jusqu'à 10% en poids d'eau, par m³ de gaz chlorhydrique – ramené aux conditions normales.

7. Procédé selon la revendication 5, caractérisé en ce que la partie supérieure de la seconde zone de lavage est chargée avec 0,01–0,5 l, de préférence 0,02–0,1 l d'acide sulfurique concentré par m³ de gaz chlorhydrique – ramené aux conditions normales.

EP 0 210 480 B1